(19) Europäisches Patentamt
European Patent Office
Office européen des brevets

(11) EP 4 506 332 A1

(12) EUROPEAN PATENT APPLICATION
published in accordance with Art. 153(4) EPC

(43) Date of publication:
**12.02.2025 Bulletin 2025/07**

(21) Application number: **23859208.3**

(22) Date of filing: **22.08.2023**

(51) International Patent Classification (IPC):
***C07C 235/34*** (2006.01) ***C07C 231/12*** (2006.01)
***A61K 31/165*** (2006.01) ***A61P 29/00*** (2006.01)
***A61P 37/00*** (2006.01)

(52) Cooperative Patent Classification (CPC):
**A61K 31/165; A61P 29/00; A61P 37/00; A61P 37/06; C07C 231/12; C07C 235/34;**
Y02A 50/30

(86) International application number:
**PCT/CN2023/114282**

(87) International publication number:
**WO 2024/046175 (07.03.2024 Gazette 2024/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.08.2022 CN 202211042237**

(71) Applicant: **Wuhan Wuyao Sci & Tech Co., Ltd**
**Wuhan, Hubei 430074 (CN)**

(72) Inventors:
- **LIU, Xianbo**
**Wuhan, Hubei 430074 (CN)**
- **ZHANG, Xiao**
**Wuhan, Hubei 430074 (CN)**
- **RAO, Xiang**
**Wuhan, Hubei 430074 (CN)**
- **LIU, Xin**
**Wuhan, Hubei 430074 (CN)**
- **CAI, Chao**
**Wuhan, Hubei 430074 (CN)**
- **LI, Lei**
**Wuhan, Hubei 430074 (CN)**
- **LI, Zhenwu**
**Wuhan, Hubei 430074 (CN)**

(74) Representative: **HGF**
**HGF Limited**
**1 City Walk**
**Leeds LS11 9DX (GB)**

# (54) PHARMACEUTICAL SALT OF POLYAMINE DERIVATIVE, CRYSTAL FORM THEREOF, AND PREPARATION METHOD THEREFOR

(57) Disclosed is a bis((N-3-ainopropyl)-(N-(3,4-dimethoxyphenylpropionyl)))aminopropylamine phosphate, a crystal form thereof, and a preparation method therefor. Compared to a free base and other salts of bis((N-3-aminopropyl)-(N-(3,4-dimethoxyphenylpropionyl)))aminopropylaine, bis((N-3-aminopropyl)-(N-(3,4-dimethoxyphenylpropionyl)))aminopropylamine phosphate has better stability and higher dynamic solubility, and is more conducive to drug safety and industrial production.

EP 4 506 332 A1

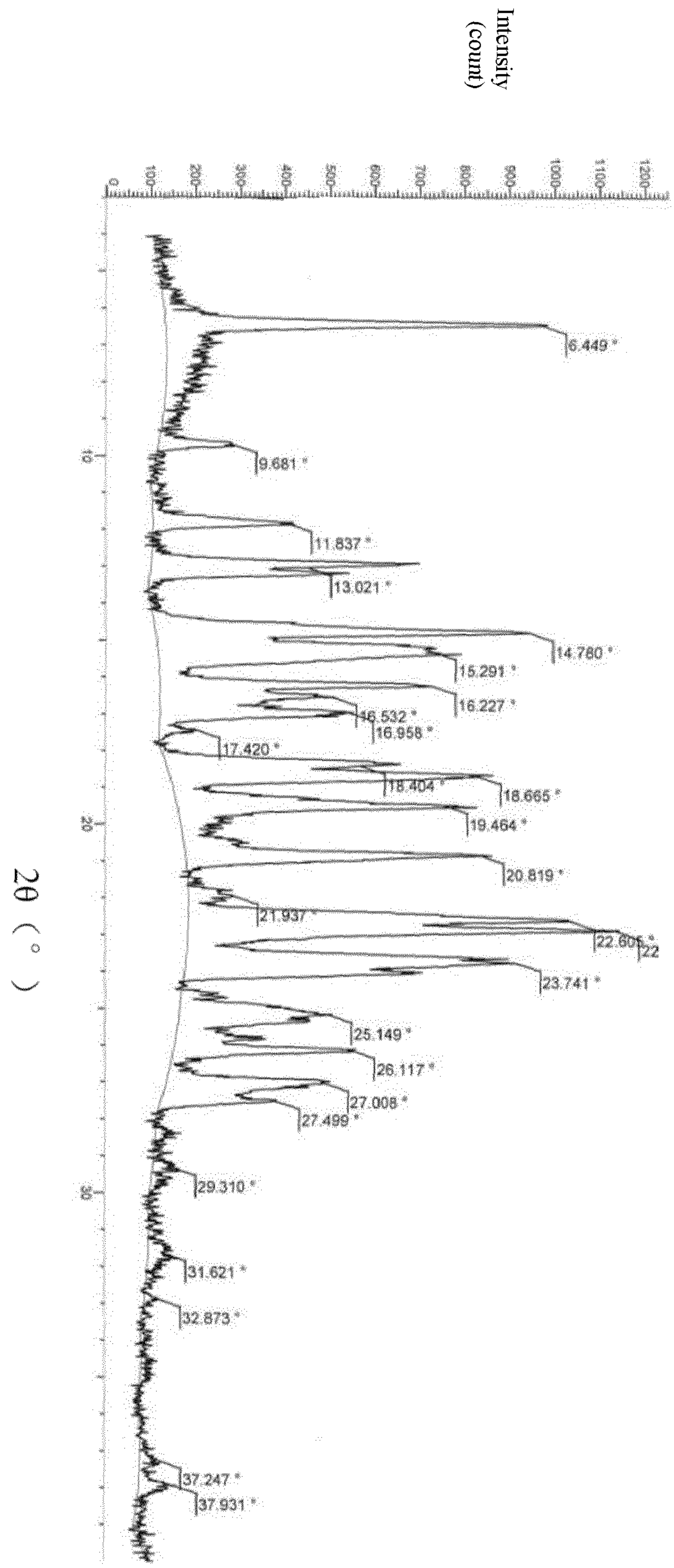
Intensity (count)
0
100
200
300
400
500
600
700
800
900
1000
1100
1200
10
20
30
2θ (°)
6.449 °
9.681 °
11.837 °
13.021 °
14.780 °
15.291 °
16.227 °
16.532 °
16.958 °
17.420 °
18.404 °
18.665 °
19.464 °
20.819 °
21.937 °
22.605 °
23.741 °
25.149 °
26.117 °
27.008 °
27.499 °
29.310 °
31.621 °
32.873 °
37.247 °
37.931 °

FIG. 1

## Description

TECHNICAL FIELD

The present invention relates to the technical field of chemical medicines, and particularly relates to a bis((N-3-aminopropyl)-(N-(3,4-dimethoxyphenylpropionyl)))aminopropylamine phosphate and a crystal form thereof and a preparation method therefor.

BACKGROUND

Systemic inflammatory response syndrome and an autoimmune disorder-related disease, such as sepsis and an autoimmune disease, are two categories of diseases caused by the body's own excessive immune response. Currently, there is still a lack of effective therapeutic medicaments, and the targeted prevention and treatment of these diseases are the focus and hot issues of clinical concern. Sepsis refers to systemic inflammatory response syndrome (SIRS) mediated by infectious factors, which affects up to 19 million people worldwide every year. Despite significant advances in current antibiotics and critical medicine technology, sepsis remains a major cause of death in infected patients and there is no ideal therapeutic medicaments to date. Studies show that the pathogenesis of sepsis lies in the fact that pathogen-associated molecular patterns (PAMPs) released by bacteria, viruses, fungi, and other pathogens are recognized by pattern recognition receptors (PRRs) of the host's innate immune system, mediating the activation of inflammatory response cells and thus causing a systemic excessive inflammatory response. Epidemiological surveys show that PAMP molecules that cause sepsis mainly include bacterial lipopolysaccharide (LPS), bacterial genome DNA (CpG DNA), peptidoglycan (PGN), lipoteichoic acid (LTA), viral RNA, and zymosan. Patent document CN105348137B discloses a pharmaceutically acceptable salt of a polyamine derivative, a preparation method therefor, and use thereof for treating sepsis, of which the pharmaceutically acceptable salt of the polyamine derivative is hydrochloride of bis((N-3-aminopropyl)-(N-(3,4-dimethoxyphenylpropionyl)))aminopropylamine with an acid-base ratio of 3. However, in actual production, it is found that the hydrochloride of bis((N-3-aminopropyl)-(N-(3,4-dimethoxyphenylpropionyl)))aminopropylamine with an acid-base ratio of 3 as disclosed in the prior art has the defects of serious wall sticking phenomenon, difficult product discharging, and poor operability during production and preparation, which is not favorable for actual production. Therefore, it is very necessary to develop a stable pharmaceutically acceptable salt of bis((N-3-aminopropyl)-(N-(3,4-dimethoxyphenylpropionyl))) aminopropylamine which is suitable for industrial production.

SUMMARY

In order to overcome the defects in the prior art, the present invention provides a bis((N-3-aminopropyl)-(N-(3,4-dimethoxyphenylpropionyl)))aminopropylamine phosphate and a crystal form thereof and a preparation method therefor.

In a first aspect of the present invention, provided is a bis((N-3-aminopropyl)-(N-(3,4-dimethoxyphenylpropionyl)))aminopropylamine phosphate.

Further, the bis((N-3-aminopropyl)-(N-(3,4-dimethoxyphenylpropionyl)))aminopropylamine phosphate has the following structure:

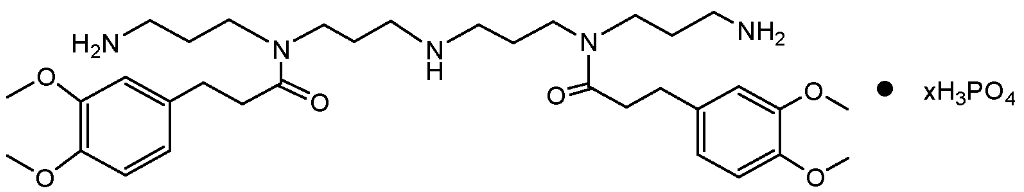

wherein x represents the number of mole(s) of phosphoric acid combined with per mole of bis((N-3-aminopropyl)-(N-(3,4-dimethoxyphenylpropionyl)))aminopropylamine; further, x is 1-3 (e.g., 1, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, and 3.0); preferably, x is 1.5-2.5; more preferably, x is 1.9-2.1; and most preferably, x is 2.0.

Preferably, the phosphate is:

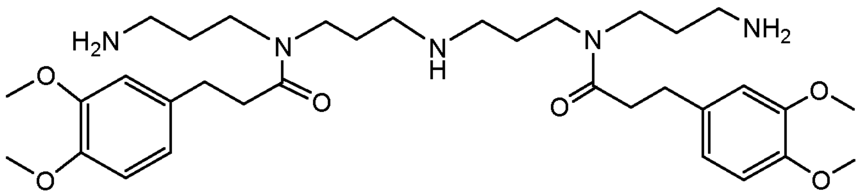

• $2H_3PO_4$

Further, the phosphate is in a crystal form, non-crystal form, or a mixed form of crystal and non-crystal.

**In** a second aspect of the present invention, provided is a preparation method for the bis((N-3-aminopropyl)-(N-(3,4-dimethoxyphenylpropionyl)))aminopropylamine phosphate according to the first aspect, wherein the preparation method comprises a step of mixing bis((N-3-aminopropyl)-(N-(3,4-dimethoxyphenylpropionyl)))aminopropylamine with phosphoric acid.

Further, the preparation method is:
mixing bis((N-3-aminopropyl)-(N-(3,4-dimethoxyphenylpropionyl)))aminopropylamine with phosphoric acid for reaction.

Further, a solvent for the reaction is selected from one or more of: ethyl acetate, dichloromethane, methanol, water, chloroform, ethanol, acetone, acetonitrile, butanol, dimethylformamide, dimethyl sulfoxide, methyl *tert-butyl* ether, isopropylamine, and isopropanol; preferably, the solvent for the reaction is selected from one or more of: methanol, ethanol, ethyl acetate, acetone, and dichloromethane; and more preferably, the solvent for the reaction is methanol or ethanol.

Further, the reaction is performed at a temperature of 0-30°C (e.g., 0°C, 5°C, 15°C, 20°C, 25°C, and 30°C), and preferably, the reaction is performed at a temperature of 0-15°C.

Further, the reaction is performed for 2-6 h (e.g., 2 h, 3 h, 4 h, 5 h, and 6 h), preferably 3-5 h, and more preferably 4 h.

Further, seed crystals are added during the reaction process.

Further, the equivalent ratio of the bis((N-3-aminopropyl)-(N-(3,4-dimethoxyphenylpropionyl)))aminopropylamine to the phosphoric acid is in the range of 1:1-1:2.5 (e.g., 1:1, 1:1.1, 1:1.2, 1:1.3, 1:1.4, 1:1.5, 1:1.6, 1:1.7, 1:1.8, 1:1.9, 1:2, 1:2.1, 1:2.2, 1:2.3, 1:2.4, and 1:2.5), preferably the equivalent ratio is in the range of 1:1-1:2, and more preferably the equivalent ratio is in the range of 1:1.8-1:2; the equivalent ratio is a molar ratio.

Further, the preparation method for the bis((N-3-aminopropyl)-(N-(3,4-dimethoxyphenylpropionyl)))aminopropylamine phosphate further comprises a step of separating the product.

Further, the separation comprises a step of filtration or centrifugation.

Further, the filtration operation refers to filtering the reaction system obtained after the reaction to obtain a filter cake, and optionally washing the filter cake.

Further, a solvent for use in the washing is selected from:one of: methanol, ethyl acetate, ethanol, and isopropanol, and preferably, the washing solvent is methanol or ethyl acetate.

Further, the preparation method for the bis((N-3-aminopropyl)-(N-(3,4-dimethoxyphenylpropionyl)))aminopropylamine phosphate further comprises a step of drying. Further, the step of drying refers to performing drying treatment under normal pressure or reduced pressure for the separated substance.

Further, the drying is performed at a temperature of 0-35°C (e.g., 0°C, 5°C, 10°C, 15°C, 20°C, 25°C, 30°C, and 35°C), and preferably, the drying is performed at a temperature of 25-35°C.

The bis((N-3-aminopropyl)-(N-(3,4-dimethoxyphenylpropionyl)))aminopropylamine may be prepared on a gram scale or a kilogram scale according to any several different methods, for example, the preparation method described in patent document CN201510729318.8, which is incorporated herein by reference.

Further, the preparation method for the bis((N-3-aminopropyl)-(N-(3,4-dimethoxyphenylpropionyl)))aminopropylamine phosphate further comprises a step of purifying (refining) the bis((N-3-aminopropyl)-(N-(3,4-dimethoxyphenylpropionyl)))aminopropylamine phosphate.

Further, the step of purification (refining) comprises recrystallization and/or washing.

Further, a solvent for the recrystallization is selected from one or a combination of two of: methanol, ethanol, and water, particularly methanol-water, ethanol-water, water-methanol, and water-ethanol. Further, a solvent for the washing is selected from one of: methanol, ethanol, ethyl acetate, and isopropanol, and preferably, the solvent for washing the crystal is methanol or ethyl acetate. Further, the step of purification (refining) comprises adding seed crystals.

In a third aspect of the present invention, provided is a preparation method for a non-crystal form of the bis((N-3-aminopropyl)-(N-(3,4-dimethoxyphenylpropionyl)))aminopropylamine phosphate according to the first aspect.

Further, the preparation method is selected from one or a combination of two or more of: a gas-liquid diffusion method, a high polymer induction method, a slow volatilization method, and a slow cooling method, and preferably, the preparation method is selected from one or a combination of two or more of: a gas-liquid diffusion method, a high polymer induction method, and a slow volatilization method.

Further, the gas-liquid diffusion method comprises the following steps: placing a target product in an open container and dissolving with a positive solvent, then placing the open container in a sealed container filled with an anti-solvent and standing (at room temperature), and finally, collecting the solid.

Further, the high polymer induction method comprises the following steps: dissolving a target product with a solvent, filtering the mixture with a syringe membrane filter, adding a high polymer to the filtrate, and volatilizing the mixture at room temperature to obtain a solid.

Further, the slow volatilization method comprises the following steps: dissolving a target product with a solvent, and then drying the mixture to obtain a solid.

Further, the slow cooling method comprises the following steps: dissolving a target product with a solvent, heating the mixture at 40-60°C, stirring and equilibrating for 1-4 h; filtering after the solution becomes clear, and slowly cooling the filtrate from 40-60°C to 0-10°C, and standing at a constant temperature of -10-30°C (e.g., 10°C, 15°C, 20°C, 25°C, and 30°C) for 3-8 days; the filtrate is still clear, and finally, subjecting to volatilization treatment at 40-60°C (e.g., 40°C, 45°C, 50°C, 55°C, and 60°C).

In one embodiment of the present invention, the preparation method for the non-crystal form of the bis((N-3-aminopropyl)-(N-(3,4-dimethoxyphenylpropionyl)))aminopropylamine phosphate is a slow volatilization method, specifically, weighing 10-20 mg of the bis((N-3-aminopropyl)-(N-(3,4-dimethoxyphenylpropionyl)))aminopropylamine phosphate in a 4 mL glass bottle, and dissolving with a mixed solution of DMSO and water at a volume ratio of 2:1, filtering with a syringe membrane filter (pore size: 0.22 $\mu$m), sealing the filtrate with Parafilm® with 5 pinholes, and slowly volatilizing the filtrate under the set temperature. The final solid obtained is characterized by XRPD. The experimental results show that amorphous bis((N-3-aminopropyl)-(N-(3,4-dimethoxyphenylpropionyl)))aminopropylamine phosphate is obtained by slow volatilization method at room temperature.

In a fourth aspect of the present invention, provided is crystal form A of the bis((N-3-aminopropyl)-(N-(3,4-dimethoxyphenylpropionyl)))aminopropylamine phosphate according to the first aspect. Further, the XRPD pattern of the crystal form A has characteristic peaks (major characteristic diffraction peaks) in at least three (or all) of positions having 2$\theta$ values of 6.4° ± 0.2°, 9.7° ± 0.2°, 16.2° ± 0.2°, 19.5° ± 0.2°, 22.9° ± 0.2°, and 26.1° ± 0.2°.

Further, the XRPD pattern of the crystal form A has characteristic peaks (major characteristic diffraction peaks) in at least three (or all) of positions having 2$\theta$ values of 6.4° ± 0.2°, 9.7° ± 0.2°, 11.80° ± 0.2°, 13.0° ± 0.2°, 14.8° ± 0.2°, 16.2° ± 0.2°, 19.5° ± 0.2°, 22.9° ± 0.2°, and 26.1° ± 0.2°. Further, the XRPD pattern of the crystal form A has characteristic peaks (secondary characteristic diffraction peaks) in at least three (at least four or all) of positions having 2$\theta$ values of 11.80° ± 0.2°, 13.0° ± 0.2°, 14.8° ± 0.2°, 15.3° ± 0.2°, 18.7° ± 0.2°, 20.8° ± 0.2°, 22.6° ± 0.2°, and 23.7° ± 0.2°. Further, the crystal form A has an XRPD pattern substantially as shown in FIG. 1.

Further, the DSC pattern of the crystal form A has an endothermic peak at 175-195°C (e.g., about 175°C, 180°C, 185°C, 190°C, and 195°C).

Further, the crystal form A has a DSC pattern substantially as shown in FIG. 2.

Further, the weight loss of the crystal form A is about 3% (e.g., 1.5%, 2.0%, 2.5%, and 3.0%) upon heating from 22°C to 150°C.

Further, the crystal form A has a TGA pattern substantially as shown in FIG. 2.

In a fifth aspect of the present invention, provided is a preparation method for the crystal form A according to the fourth aspect.

Further, the preparation method is an anti-solvent crystallization method and an anti-anti-solvent crystallization method.

Further, the anti-solvent crystallization method comprises the following steps: dissolving a target product with a positive solvent, and then adding an anti-solvent into the system, wherein the anti-solvent can not dissolve or only slightly dissolve the substance to be crystallized, thus reducing the solubility of the crystallized substance and precipitating it from the mixed solution.

Further, the anti-anti-solvent crystallization method comprises: dissolving a solution of a target product with a positive solvent, and adding the solution into one or more anti-solvents, so that the product is in a slightly soluble state in the solution, thereby precipitating crystals after the solution reaches a supersaturated state.

In one embodiment of the present invention, the anti-solvent crystallization method for the crystal form A of bis((N-3-aminopropyl)-(N-(3,4-dimethoxyphenylpropionyl))) aminopropylamine phosphate specifically comprises:

(1) mixing the reaction product obtained in the second aspect of the present invention with a positive solvent;
(2) dropwise adding an anti-solvent into the mixed solution obtained in step (1);
(3) cooling the solution obtained in step (2), and separating to obtain a wet product; and
(4) drying the wet product obtained in step (3) to obtain a dry product of phosphate crystal A. Further, the positive solvent in step (1) is selected from: water, a mixed solution of methanol and water, or a mixed solution of ethanol and water; and preferably, the positive solvent in step (1) is a mixed solution of methanol and water.

Further, the anti-solvent in step (1) is selected from one or more of: methanol, ethanol, tetrahydrofuran, ethyl acetate, or toluene.

Further, the cooling in step (3) refers to reducing the temperature to 0-15°C (e.g., 0°C, 5°C, 10°C, and 15°C), and preferably, the cooling in step (3) refers to reducing the temperature to 5°C.

Further, the cooling in step (3) is performed for 3-16 h (e.g., 3 h, 4 h, 5 h, 6 h, 7 h, 8 h, 9 h, 10 h, 11 h, 12 h, 13 h, 14 h, 15 h, and 16 h); preferably, the cooling in step (3) is performed for 8-14 h (e.g., 8 h, 9 h, 10 h, 11 h, 12 h, 13 h, and 14 h); and further preferably, the cooling in step (3) is performed for 12 h.

Further, the separation method in step (3) comprises centrifugation, filtration, and/or vacuum filtration. Preferably, the centrifugation refers to centrifuging the mixed solution after the reaction to obtain a solid.

Further, the drying in step (4) is performed at a temperature of 25-35°C (e.g., 25°C, 30°C, and 35°C), and preferably, the drying is performed at a temperature of 30°C.

In a six aspect of the present invention, provided is use of the bis((N-3-aminopropyl)-(N-(3,4-dimethoxyphenylpropionyl)))aminopropylamine phosphate and crystal form A thereof in preparing an anti-PAMP medicament.

Further, the PAMP is selected from one or more of: bacterial lipopolysaccharide (LPS), bacterial genome DNA (CpG DNA), peptidoglycan (PGN), lipoteichoic acid (LTA), viral RNA, and zymosan. In a seventh aspect of the present invention, provided is use of the bis((N-3-aminopropyl)-(N-(3,4-dimethoxyphenylpropionyl)))aminopropylamine phosphate and crystal form A thereof in preparing a medicament for preventing and/or treating systemic inflammatory response syndrome (SIRS).

Further, the systemic inflammatory response syndrome is sepsis.

In an eighth aspect of the present invention, provided is use of the bis((N-3-aminopropyl)-(N-(3,4-dimethoxyphenylpropionyl)))aminopropylamine phosphate and crystal form A thereof in preparing a medicament for preventing and/or treating an autoimmune disease.

Further, the autoimmune disease is selected from one or more of: organ-specific autoimmune disease, systemic lupus erythematosus, rheumatoid arthritis, systemic vasculitis, scleroderma, pemphigus, dermatomyositis, mixed connective tissue disease, autoimmune hemolytic anemia, thyroid autoimmune disease, and ulcerative colitis.

Compared to free bases and other salts of bis((N-3-aminopropyl)-(N-(3,4-dimethoxyphenylpropionyl)))aminopropylamine, the bis((N-3-aminopropyl)-(N-(3,4-dimethoxyphenylpropionyl)))aminopropylamine phosphate has better stability and higher dynamic solubility, and is conducive to industrial production, wherein bis((N-3-aminopropyl)-(N-(3,4-dimethoxyphenylpropionyl)))aminopropylamine diphosphate is more stable than bis((N-3-aminopropyl)-(N-(3,4-dimethoxyphenylpropionyl)))aminopropylamine triphosphate, which is more conducive to medication safety. By adopting the phosphoric acid equivalent ratio according to the present application, in the preparation process of the bis((N-3-aminopropyl)-(N-(3,4-dimethoxyphenylpropionyl)))aminopropylamine phosphate, the salt can be easily precipitated from the reaction system, and can be separated from the system by simple filtration operation, which is greatly conducive to industrial mass production.

## BRIEF DESCRIPTION OF THE DRAWINGS

FIG. 1 shows the XRPD pattern of the phosphate crystal form A.
FIG. 2 shows the TGA/DSC pattern of the phosphate crystal form A.

## DETAILED DESCRIPTION

Some of the abbreviations used in the present invention are explained as follows:

XRPD: X-ray powder diffraction
DSC: differential scanning calorimetry
TGA: thermogravimetric analysis

In the present invention, the term "crystal form" is confirmed by X-ray powder diffraction pattern characterization. Those skilled in the art can appreciate that the physicochemical properties discussed herein can be characterized with experimental error depending on the conditions of the instrument, sample preparation, purity of the sample, etc. In particular, it is well known to those skilled in the art that the X-ray diffraction pattern will generally vary with the conditions of the instrument. It is particularly noted that the relative intensities of the X-ray powder diffraction patterns may also vary with the experimental conditions, so that the order of the peak intensities cannot be the only or decisive factor. Indeed, the relative intensities of the diffraction peaks in the XRPD pattern are related to the preferred orientation of the crystals, and the peak intensities shown herein are illustrative and not for absolute comparison. In addition, experimental errors in peak

angles are typically 5% or less, and these angles should also be taken into account, typically allowing errors of ± 0.2°. In addition, due to the influence of experimental factors such as sample thickness, an overall shift in peak angle is caused, and a certain shift is usually allowed. Thus, it can be understood by those skilled in the art that the X-ray powder diffraction pattern of a crystal form of the present invention is not required to be identical to that of the examples referred to herein, that the "XRPD patterns are identical" described herein does not mean absolutely identical, and that the identical peak positions may differ by ± 0.2° and a certain variability in peak intensities is permitted. Any crystal form having the same or similar pattern as the characteristic peaks in these patterns falls within the scope of the present invention. Those skilled in the art can compare the patterns listed in the present invention with a pattern of an unknown crystal form to confirm whether the two sets of patterns reflect the same or different crystal forms. In some embodiments, the crystal form A of the present invention is pure, single, and substantially free of any other crystal forms in admixture. In the present invention, "substantially free", when used in reference to a novel crystal form, means that the crystal form contains less than 20% by weight of other crystal forms, in particular less than 10% by weight of other crystal forms, more particularly less than 5% by weight of other crystal forms, and even more particularly less than 1% by weight of other crystal forms.

It needs to be understood that the numerical values and ranges of numerical values set forth herein should not be construed narrowly as to the numerical values or ranges themselves and that those skilled in the art should recognize that the numerical values and ranges of numerical values may be varied around specific numerical values in different technical environments without departing from the spirit and principle of the present invention. In the present invention, the floating range, which can be foreseen by those skilled in the art, is mostly expressed by the term "about". When the term "about" is used in front of a numerical value of the present invention and refers to the numerical value, it means any value within a range of ± 10%, preferably within a range of ± 5%, more preferably within a range of ± 2%, more preferably within a range of ± 1%, of the value. For example, "about 10" should be interpreted to mean 9-11, preferably 9.5-10.5, more preferably 9.8-10.2, and more preferably 9.9-10.1.

It needs to be understood that in the X-ray diffraction pattern of the powder sample, the particular crystal form of the diffraction pattern obtained from a crystalline compound is often characteristic, wherein the relative intensities of the bands (especially at low angles) may vary due to the preferential orientation effects resulting from differences in crystallization conditions, particle sizes, relative content of mixtures, and other test conditions. Therefore, the relative intensities of the diffraction peaks are not characteristic for the crystal concerned, and in the case of judging whether the crystals are identical to the known crystal forms, the positions of the peaks should be noted rather than the relative intensities thereof.

In the present invention, the term "substantially as shown in FIG...." means that at least 50%, at least 60%, at least 70%, at least 80%, at least 90%, at least 95%, or at least 99% of the peaks in the XRPD pattern, the DSC pattern, or the TGA pattern are shown in the figure.

In the present invention, the term "room temperature" means that the temperature of an article is close to or the same as the temperature of a space (e.g., the location of a fume hood in which the article is located). Typically, the room temperature is about 20°C to about 30°C, or about 22°C to 27°C, or about 25°C.

The anti-solvent crystallization (also called anti-solvent addition, precipitation crystallization, salting out, or crystallization) method is generally a method comprising precipitating crystals after bringing a solution into a supersaturated state by adding one or more anti-solvents to the solution in which a target product is dissolved with a positive solvent, the product being in a slightly soluble state in the solution. The anti-anti-solvent crystallization method is generally a method comprising precipitating crystals after bringing a solution into a supersaturated state by adding one or more anti-solvents to the solution in which a target product is dissolved with a positive solvent, the product being in a slightly soluble state in the solution.

The capability of an anti-solvent for dissolving a target product is poorer than that of a positive solvent, e.g., by more than 10%, 20%, 30%, 40%, 50%, 60%, 70%, or 80%, and therefore, the anti-solvent in a system is a relative term. The positive solvent and the anti-solvent may be polar or nonpolar solvents, and for example, may be selected from: one or more of dimethylformamide (DMF), dimethyl sulfoxide (DMSO), water, alcoholic solvents, ether solvents, ketone solvents, ester solvents, alkane solvents, aromatic solvents, and nitrile solvents, wherein the alcoholic solvents include, but are not limited to, methanol, ethanol, propanol, isopropanol or 1,3-propanediol, 1,2-propanediol or chlorobutanol or a combination thereof; ether solvents include, but are not limited to, such as tetrahydrofuran, methyl *tert*-butyl ether or 1,4-dioxane or a combination thereof; ketone solvents include, but are not limited to, acetone, methyl ethyl ketone or 4-methyl-2-pentanone or a combination thereof; ester solvents include, but are not limited to, ethyl acetate, isopropyl acetate, *n*-butyl acetate or *tert*-butyl acetate or a combination thereof; alkane solvents include, but are not limited to, dichloromethane, chloroform, *n*-hexane, cyclohexane or pentane or *n*-heptane or a combination thereof; aromatic solvents include, but are not limited to, benzene and toluene or a combination thereof; nitrile solvents include, but are not limited to, acetonitrile and malononitrile. Anti-solvent crystallization and anti-anti-solvent crystallization may be performed by batch, semi-batch, or continuous crystallization operations. The addition of an anti-solvent to a solution (anti-solvent crystallization) or the addition of a product solution to an anti-solvent (anti-anti-solvent crystallization) may either be dropwise addition at a constant rate or be dropwise addition slowly at the beginning and then gradually increasing in rate.

The disclosures of the various publications, patents, and published patent specifications cited herein are hereby incorporated by reference in their entireties.

The technical solutions of the present invention will be clearly and completely described below with reference to the examples of the present invention, and it is obvious that the described examples are only a part of the examples of the present invention but not all of them. Based on the examples of the present invention, all other examples obtained by those of ordinary skills in the art without creative work shall fall within the protection scope of the present invention.

The instrument information and methods used in the experiments of the examples are as follows:

1. X-ray powder diffraction (XRPD)

The conditions of the X-ray powder diffractometer (XRPD) test were as follows: X-ray powder diffraction data of a sample(s) was collected at ambient conditions using Bruker D2 PHASER X-ray powder diffractometer with an X-ray emitter power of 300 W, a sample stage with no background signal, a step speed of 0.15 s/step, total step of 1837 steps, a step size of 2θ=0.02°, a voltage of 30 kV, and a current of 10 mA. The X-ray tube was a tube of Cu target (Kα) with a Kα2/Kα1 strength ratio of 0.50 (1.54439 Å/1.5406 Å).

2. Thermogravimetric analysis (TGA) and differential scanning calorimetry (DSC)

Thermogravimetric data of phosphate(s) was collected by using a TA Discovery-series thermogravimetric analyzer (TGA). Several milligrams of a sample were placed in a Tzero aluminum pan and heated from room temperature to the target temperature under $N_2$ atmosphere, with an $N_2$ flow rate of 25 mL/min and a heating rate of 10°C/min. Thermal data of a sample(s) was collected by using a TADiscovery-series differential scanning calorimeter (DSC). For a crystal form sample, several milligrams of the sample were weighed and placed in a Tzero aluminum pan, sealed with a Tzero sealing lid, and heated under $N_2$ atmosphere with an $N_2$ flow rate of 50 mL/min and a heating rate of 10°C/min. For an amorphous sample, the modulation mode was set for testing: about 10 mg of the sample was weighed and placed in a Tzero aluminum pan, sealed with a Tzero sealing lid, and heated under $N_2$ atmosphere with an $N_2$ flow rate of 50 mL/min. The modulation temperature amplitude was ± 1°C, the modulation period was 40 s, the heating rate was 1°C/min, and the range of the test temperature was 25-200°C.

3. High performance liquid chromatography (HPLC)

The stability of a sample(s) was determined by HPLC, and specifically, Agilent 1260 Infinity II LC System (equipped with a DAD detector) was used to collect the purity and solubility data of the sample(s). The test method is shown in Table 1.

Table 1

| Item | Parameter | |
|---|---|---|
| Instrument | Agilent 1260 Infinity II LC System (DAD detector) | |
| Chromatographic column | Kinetex® XB-C18 4.6*250mm, 5 μm PN: 00G-4605-E0 | |
| Mobile phase | A: 10 mM ammonium acetate (adjusted to pH 2.2 with acetic acid) | B: methanol |
| Detector | DAD, wavelength: 230 nm | |
| Column temperature | 40 °C | |
| Flow rate | 1.0 mL/min | |
| Injection volume | 5 μL | |
| Diluent | 50% ACN | |
| Sample concentration | 1 mg/mL | |

(continued)

| | Time (min) | %A | %B |
|---|---|---|---|
| Elution procedure | 0 | 90 | 10 |
| | 6 | 60 | 40 |
| | 18 | 30 | 70 |
| | 22 | 10 | 90 |
| | 32 | 10 | 90 |
| | 32.1 | 90 | 10 |
| | 40 | 90 | 10 |

4. Content of phosphate radical ion

For bis((N-3-aminopropyl)-(N-(3,4-dimethoxyphenylpropionyl)))aminopropylamine phosphate, data of phosphate radical ion content of a sample(s) was collected using a DIONEX ICS-6000+ DP ion chromatographic system. The test method is shown in Table 2.

Table 2

| Item | Parameter |
|---|---|
| Reagent | Purified water (Milli-Q water)<br>Tripotassium phosphate (AR grade, Taitan)<br>Potassium hydroxide (Eluent Generator 500 KOH, PN: 075778) |
| Chromatographic column | DionexIonPac AS11-HC, 4×250mm, Part No. 052960<br>Guard column: DionexIonPac AG11-HC, 4×50mm, Part No. 052962 |
| Column temperature | 30 °C |
| Flow rate | 1.0 mL/min |
| Detector | Electrical conductivity detector |
| Suppressor | ASRS 300 4mm, Part No.SP6949 |
| Mobile phase | A: purified water, isocratic elution: 30 mM potassium hydroxide |
| Injection volume | 25 μL |
| Run time | 15 min |

5. Preparation of biological vehicles

The specific procedures for the preparation of biological vehicles are shown in Table 3.

Table 3

| Biological vehicle | Preparation method |
|---|---|
| Simulated fasting intestinal juice FaSSIF | Weighing 210 mg of NaOH, 1975 mg of $NaH_2PO_4 \cdot 2H_2O$, and 3095 mg of NaCl into a 500 mL beaker, and dissolving by adding 450 mL of deionized water;<br>adjusting the pH to 6.5 with 1 N NaOH or 1 N HCl; and fixing the volume up to 500 mL with deionized water to obtain FaSSIF blank vehicle.<br>weighing 112 mg of SIF powder into 25 mL of the FaSSIF blank vehicle described above, and stirring until completely dissolved; fixing the volume up to 50 mL with the FaSSIF blank vehicle to obtain vehicle FaSSIF;<br>standing at room temperature for 2 h before use. |

(continued)

| Biological vehicle | Preparation method |
|---|---|
| Simulated feeding intestinal juice FeSSIF | Weighing 2020 mg of NaOH, 4325 mg of glacial acetic acid, and 5935 mg of NaCl into a 500 mL beaker, and dissolving by adding 450 mL of deionized water;<br>adjusting the pH to 5.0 with 1 N NaOH or 1 N HCl, and fixing the volume up to 500 mL with deionized water to obtain FeSSIF blank vehicle;<br>weighing 280 mg of SIF powder into 15 mL of the FeSSIF blank vehicle described above, and stirring until dissolved; fixing the volume up to 25 mL with the FeSSIF blank vehicle to obtain vehicle FeSSIF;<br>standing at room temperature before use, and ensuring that it is a clear solution. |
| Simulated gastric juice SGF | Weighing 1000 mg of NaCl into a 500 mL beaker, and dissolving by adding 450 mL of deionized water;<br>adjusting the pH to 1.6 with 1 N HCl, and fixing the volume up to 500 mL with deionized water to obtain SGF blank vehicle;<br>weighing 6 mg of SIF powder into 50 mL of the SGF blank vehicle described above, and stirring until dissolved; fixing the volume up to 100 mL with the SGF blank vehicle to obtain vehicle SGF;<br>standing at room temperature before use, and ensuring that it is a clear solution. |

6. Determination of hygroscopicity

The hygroscopicity was determined by referring to the guiding principle of the hygroscopicity test of medicaments in the Phamacopoeia of the People's Republic of China 2015 Editi*on.* The specific test method is as follows:

1. taking a dried glass weighing bottle with a stopper (outside diameter: 50 mm, height: 15 mm) and placing in a suitable 25°C ± 1°C thermostatic dryer (with a saturated ammonium chloride or ammonium sulfate solution placed in the lower part) or a climatic cabinet (with temperature set at 25°C ± 1°C, and relative humidity set at 80% ± 2%) one day before the test, and accurately weighing the weight(m1).

2. taking an appropriate amount of a test sample, spreading it in the weighing bottle described above with a thickness of about 1 mm, and accurately weighing the weight (m2).

3. opening the weighing bottle, and placing it under constant temperature and humidity conditions for 24 h together with the bottle cap.

4. covering the weighing bottle, and accurately weighing the weight (m3).

$$\text{Percentage of weight gain} = (m3\text{-}m2)/(m2\text{-}m1) \times 100\%$$

5. describing the characteristics of hygroscopicity and defining hygroscopic weight gain

Deliquescence: Sufficient water is absorbed to form a liquid.
Extremely hygroscopic: The hygroscopic weight gain is not less than 15%.
Hygroscopic: The hygroscopic weight gain is less than 15% but not less than 2%.
Slightly hygroscopic: The hygroscopic weight gain is less than 2% but not less than 0.2%. Non-hygroscopic or almost non-hygroscopic: The hygroscopic weight gain is less than 0.2%.

**Example 1**

**Screening of salt form**

A solution of bis((N-3-aminopropyl)-(N-(3,4-dimethoxyphenylpropionyl)))aminopropylamine free base (hereinafter referred to as free base) in ethanol was prepared at room temperature. An acid solution was weighed into an HPLC vial according to an acid-base molar feeding ratio of 1:1, the solution of free base in ethanol was added thereto, and the resulting mixture was magnetically stirred at room temperature. After the mixture was magnetically stirred for 2-4 days at room temperature, a few systems gave turbid solids, phenomena of wall sticking and gelling were observed in some

systems, and the rest systems were still clear. The subsequent processing steps were as follows: 1) the turbid system(s) was centrifuged to separate solids; 2) the wall-sticking and gelling system(s) was subjected to cyclic heating and cooling treatment at 25-50°C (the heating and cooling procedure is shown in Table 5) for the purpose of trying to improve the apparent state/obtain solids; 3) the clear or slightly turbid system(s) was magnetically stirred at 5°C for about 5 h, and then left to stand at - 20°C overnight for the purpose of trying to collect a sufficient amount of solids for subsequent characterization, and if the amount of solids was still low, slow volatilization at room temperature was performed; if the system(s) was still clear ultimately, anti-solvent addition (n-heptane) or slow volatilization at room temperature was performed. The solids obtained above were centrifuged. The test results are shown in Table 4.

Table 4

| | Acids | Salt-forming morphology |
|---|---|---|
| 1 | Hydrochloric acid$^{\alpha}$ | Gel* |
| 2 | Sulfuric acid$^{\alpha}$ | Solid |
| 3 | Phosphoric acid$^{\alpha}$ | Solid |
| 4 | p-Toluene sulfonic acid | Gel* |
| 5 | Methanesulfonic acid | Gel* |
| 6 | Oxalic acid | Solid |
| 7 | Maleic acid | Gel# |
| 8 | Gentisic acid | Gel† |
| 9 | L-tartaric acid | Solid ※ |
| 10 | Fumaric acid | Gel# |
| 11 | Hippuric acid | Oil# |
| 12 | Benzoic acid | Oil# |
| 13 | Succinic acid | Gel* |
| 14 | Hydrochloric acid$^{\beta}$ | Gel# |
| 15 | Sulfuric acid$^{\beta}$ | Gel& |
| 16 | Phosphoric acid$^{\beta}$ | Solid |

$\alpha$: The acid-base molar feeding ratio was 1:1.

$\beta$: The acid-base molar feeding ratio was 3:1.

*: The solid content was less or the system was clear, and slow volatilization at room temperature (18-22°C) was performed after the treatment at a low temperature of 5°C/-20°C.

#: The solid content was less or the system was clear, and anti-solvent addition was performed after the treatment at a low temperature of 5°C/-20°C.

†: The system was gelled, and the state was not improved after cyclic heating and cooling treatment at 25-50°C.

&: The reaction gave a small amount of solids, but the system was gelled after vacuum drying at 30°C for 17 h.

※: The solids obtained from the reaction were gelled after being sealed and placed at room temperature for 1 week.

Table 5

| Serial No. | Temperature (°C) | Temperature rising and falling rate (°C/min) | Isothermal time (h) |
|---|---|---|---|
| 1 | 25 | 0 | 0.3 |
| 2 | 25-50 | 0.4 | 1 |
| 3 | 50-35 | 0.1 | 1 |
| 4 | 35-50 | 0.25 | 0.5 |
| 5 | 50-20 | 0.1 | 4~18 |

**Stability test**

To assess the solid stability of different salts, about 15 mg of a sample was weighed into an HPLC vial, and the vial was opened and placed at 25°C/60% RH. The initial sample and the samples stored for 5 days and 2 weeks were subjected to chemical purity assays (HPLC) and hygroscopicity tests. The stability data are summarized in Table 6. The results showed that the phosphate prepared with an acid-base feeding ratio of 1 had no obvious change in properties and purity after being placed under the selected conditions for 5 days and 2 weeks, while the hydrochloride, sulfate, and oxalate prepared with an acid-base feeding ratio of 1 and the phosphate prepared with an acid-base feeding ratio of 3 were observed to change into liquid from powdery solid on the fourth day and had a certain degree of purity reduction; the phosphate prepared with an acid-base feeding ratio of 1 had a hygroscopicity value of 1.29%, which was slightly hygroscopic; and other samples had a hygroscopicity value of more than 15%, which were extremely hygroscopic. The above experimental data showed that only the phosphate prepared with an acid-base feeding ratio of 1 was relatively stable among the five salt forms. The solid phosphate prepared with an acid-base feeding ratio of 1 or 3 was sampled to measure the acid-base ratio, and the test method for the content of phosphate radical ions is shown in Table 2. The test results showed that the acid-base ratio of the phosphate prepared with an acid-base feeding ratio of 1 was 1.9, and the acid-base ratio of the phosphate prepared with an acid-base feeding ratio of 3 was 3.0.

Table 6

| Stability | | Hydrochloride$^{\alpha}$ | Sulfate$^{\alpha}$ | Phosphoric acid$^{\alpha}$ (acid-base ratio of 1.9) | Oxalate | Phosphoric acid$^{\alpha}$ (acid-base ratio of 3.0) |
|---|---|---|---|---|---|---|
| Purity (peak area, %) | 0 day | 99.49 | 99.75 | 99.56 | 99.53 | 99.45 |
| | 5 days | 99.26 | 99.61 | 99.55 | 99.33 | 99.41 |
| | 2 weeks | 98.81 | 99.47 | 99.46 | 99.10 | 99.24 |
| Hygroscopicity (%) | | 24.7 | 28.29 | 1.29 | 18.62 | 18.05 |

The stability of the samples were determined by HPLC, and specifically, Agilent 1260 Infinity II LC System (equipped with a DAD detector) was used to collect the purity and solubility data of samples. The test method is shown in Table 1. The hygroscopicity of samples was determined according to the method in the *Phamacopoeia of the People's Republic of China,* and the specific determination method is shown in section "6. Determination of hygroscopicity" of the specification.

**Dynamic solubility**

The phosphate was subjected to dynamic solubility tests in water and 3 biological vehicles (simulated gastric juice SGF, simulated fasting intestinal juice FaSSIF, and simulated feeding intestinal juice FeSSIF) at 37°C, and the specific method for the solubility tests were as follows: four samples of 30 mg each were weighed, and added into 3 mL of water and 3 biological solvents separately, and shaken at a constant temperature of 37°C (100 rpm); and 1.0 mL of the suspension (or clear solution) was taken at 1/4/24 h separately and centrifuged for 3 min, and the supernatant/clear solution was filtered, and then subjected to solubility tests (HPLC) and pH determination (pH meter). The specific procedure for the preparation of the biological vehicles is shown in Table 3.

The solubility of the phosphate in water/SGF/FaSSIF/FeSSIF was >9 mg/mL. The solubility of samples was determined by HPLC, and specifically, Agilent 1260 Infinity II LC System (equipped with a DAD detector) was used to collect the purity and solubility data of samples. The test method is shown in Table 1 above. The pH value did not change within 24 h. The phosphate had good solubility both in water and simulated *in-vivo* environment.

Conclusion: unlike the bis((N-3-aminopropyl)-(N-(3,4-dimethoxyphenylpropionyl)))aminopropylamine phosphate, the bis((N-3-aminopropyl)-(N-(3,4-dimethoxyphenylpropionyl)))aminopropylamine free base has lower stability, and a purity reduced from 97.56% to 73.73% after left at room temperature for 7 days, and thus needs to be stored at -20°C. The bis((N-3-aminopropyl)-(N-(3,4-dimethoxyphenylpropionyl)))aminopropylamine hydrochloride is extremely hygroscopic, and present in colloidal form at room temperature, and it is impossible to obtain a sample with good solid properties without vacuum drying. The sulfate prepared with an acid-base feeding ratio of 1 can form a solid at room temperature, but the formed salt has poor stability at room temperature and is extremely hygroscopic. The L-tartrate can form a solid at room temperature, but the obtained solid was gelled after being sealed and placed at room temperature for 1 week. The oxalate and the phosphate prepared with an acid-base feeding ratio of 3 are extremely hygroscopic with poor stability. Due to the extremely hygroscopic property, different degrees of wall sticking will occur in the preparation process,

which is not conducive to industrial production.

The phosphate prepared with an acid-base feeding ratio of 1 has a good suspension state in the salt-forming process, and the salt can be easily precipitated and separated from the system. The phosphate prepared has good stability and no obvious change in properties and purity after being placed for two weeks, with relatively lower hygroscopicity, which is greatly conducive to industrial mass production.

## Preparation of salt

### Example 2

To a 100 mL three-neck flask, at room temperature, bis((N-3-aminopropyl)-(N-(3,4-dimethoxyphenylpropionyl))) aminopropylamine free base (2 g) was added, and then dichloromethane (20 g) was added. The mixture was stirred for dissolution and became clear to obtain a solution of the free base in dichloromethane. The solution was cooled to 0-5°C.

To a centrifuge tube, phosphoric acid (695 mg, 85%) was added, and then ethyl acetate (10 g) was added to obtain a solution of phosphoric acid in ethyl acetate, which was shaken well for later use. The solution of phosphoric acid in ethyl acetate was slowly and dropwise added to the solution of the free base in dichloromethane. The mixture was reacted under stirring at 0-5°C for 4 h, and a white solid was gradually precipitated. The system became turbid and conglobulated, and became a white suspension in the stirring process. The mixture was filtered, and the filter cake was washed with ethyl acetate to obtain a wet product. The solid was dried in an oven at 30°C for 16 h to obtain a white solid (2.1 g). The solid was sampled for purity, content, and acid-base ratio tests, and the test results are shown in Table 7.

### Example 3

To a 100 mL three-neck flask, at room temperature, bis((N-3-aminopropyl)-(N-(3,4-dimethoxyphenylpropionyl))) aminopropylamine free base (1 g) was added, and then methanol (10 g) was added. The mixture was stirred for dissolution and became clear to obtain a solution of the free base in methanol. The solution was cooled to 0-5°C.

To a centrifuge tube, phosphoric acid (347.8 mg, 85%) was added, and then methanol (5 g) was added to obtain a solution of phosphoric acid in methanol, which was shaken well for later use.

The solution of phosphoric acid in methanol was slowly and dropwise added to the solution of the free base in methanol. The mixture was reacted under stirring at 0-5°C for 4 h. The system was in a clear state at the beginning and gradually became turbid after 0.5 h, and then, a large amount of solids were precipitated. The mixture was filtered, and the filter cake was washed with ethyl acetate to obtain a wet product. The solid was dried in an oven at 30°C for 16 h to obtain a white solid (1.1 g). The solid was sampled for purity, content, and acid-base ratio tests. The test results are shown in Table 7.

### Example 4

To a 100 mL three-neck flask, at room temperature, bis((N-3-aminopropyl)-(N-(3,4-dimethoxyphenylpropionyl))) aminopropylamine free base (5 g) was added, and then methanol (50 g) was added. The mixture was stirred for dissolution and became clear to obtain a solution of the free base in methanol. The solution was cooled to 0-5°C.

To a 50 mL beaker, phosphoric acid (1.65 g, 85%) was added, and then methanol (25 g) was added to obtain a solution of phosphoric acid in methanol, which was shaken well for later use.

The solution of phosphoric acid in methanol was slowly and dropwise added to the solution of the free base in methanol. The mixture was reacted under stirring at 0-5°C for 4 h. The system was in a clear state at the beginning and gradually became turbid after 0.5 h, and then, a large amount of solids were precipitated. The mixture was filtered, and the filter cake was washed with methanol to obtain a white wet product. The solid was dried in an oven at 30°Cfor 16 h to obtain a white solid (5.5 g). The solid was sampled for purity, content, and acid-base ratio tests, and the test results are shown in Table 7.

### Example 5

To a 100 mL three-neck flask, at room temperature, bis((N-3-aminopropyl)-(N-(3,4-dimethoxyphenylpropionyl))) aminopropylamine free base (3.4 g) was added, and then methanol (34 g) was added. The mixture was stirred for dissolution and became clear to obtain a solution of the free base in methanol. The solution was cooled to 0-5°C.

To a 50 mL beaker, phosphoric acid (1.12 g, 85%) was added to a 50 mL beaker, and methanol (17 g) was added to obtain a solution of phosphoric acid in methanol. The solution was shaken well for later use.

The solution of phosphoric acid in methanol was slowly and dropwise added to the solution of the free base in methanol. The mixture was reacted under stirring at 0-5°C for 4 h. The system was in a clear state at the beginning and

gradually became turbid after 0.5 h, and then, a large amount of solids were precipitated. The mixture was filtered, and the filter cake was washed with methanol to obtain a white wet product. The solid was dried in an oven at 30°C for 16 h to obtain a white solid (5.5 g). The solid was sampled for purity, content, and acid-base ratio tests, and the test results are shown in Table 7.

**Example 6**

To a 100 mL three-neck flask, at room temperature, bis((N-3-aminopropyl)-(N-(3,4-dimethoxyphenylpropionyl))) aminopropylamine free base (1.0 g) was added, and then methanol (10 g) was added. The mixture was stirred for dissolution and became clear to obtain a solution of the free base in methanol. The solution was cooled to 0-5°C.

To a centrifuge tube, phosphoric acid (183 mg, 85%) was added, and then methanol (5 g) was added to obtain a solution of phosphoric acid in methanol, which was shaken well for later use.

The solution of phosphoric acid in methanol was slowly and dropwise added to the solution of the free base in methanol. The mixture was reacted under stirring at 0-5°C for 4 h. The mixture was filtered, and the filter cake was washed with methanol to obtain a white wet product. The solid was dried in an oven at 30°Cfor 16 h to obtain a white solid (0.8 g). The solid was sampled for purity, content, and acid-base ratio tests, and the test results are shown in Table 7.

Researchers found that the phosphate prepared with an acid-base ratio of about 2 has almost unchanged acid-base ratio after recrystallization in methanol-water or water-methanol recrystallization system, which was stable in the recrystallization process.

**Comparative Example 1**

To a 100 mL three-neck flask, at room temperature, bis((N-3-aminopropyl)-(N-(3,4-dimethoxyphenylpropionyl))) aminopropylamine free base (1.0 g) was added, and then methanol (10 g) was added. The mixture was stirred for dissolution and became clear to obtain a solution of the free base in methanol. The solution was cooled to 0-5°C.

To a centrifuge tube, phosphoric acid (558.3 mg, 85%) was added, and then methanol (5 g) was added to obtain a solution of phosphoric acid in methanol which was shaken well for later use.

The solution of phosphoric acid in methanol was slowly and dropwise added to the solution of the free base in methanol. The mixture was reacted under stirring at 0-5°C for 4 h. Serious wall sticking was observed. After the methanol was concentrated to dryness, the sticky solid on the wall was scraped by a spatula, slurried with ethyl acetate, and then filtered. After the filtration was completed, the solid was extremely hygroscopic and quickly turned into an oil. The oil was dried in an oven at 30°C for 16 h to obtain a solid. The solid was sampled for purity, content, and acid-base ratio tests. The acid-base ratio is the molar amount of phosphoric acid/the molar amount of bis((N-3-aminopropyl)-(N-(3,4-dimethoxyphenylpropionyl)))aminopropylamine, namely the acid-base molar ratio. The test method for the content of phosphate radical ions is shown in Table 2, and the test results are shown in Table 7.

Researchers found that the acid-base ratio of the phosphate having an acid-base ratio of 3 prepared was reduced by more than 10% after recrystallization in methanol-water or water-methanol recrystallization system, that is, the phosphate having an acid-base ratio of 3 prepared with a phosphoric acid equivalent ratio of 3.05 eq was unstable.

Table 7

<table>
<tr><th>Examples</th><th>Feeding amount (free base)</th><th>Phosphoric acid equivalent number</th><th>Acid-base ratio</th><th>Purity</th><th>Weight of phosphate</th><th>Purity of free base</th></tr>
<tr><td>Example 2</td><td>2 g</td><td>1.9 eq</td><td>2.1</td><td>99.12%</td><td>2.1 g</td><td>97.45%</td></tr>
<tr><td>Example 3</td><td>1 g</td><td>1.8 eq</td><td>2.0</td><td>98.83%</td><td>1.1 g</td><td rowspan="2">97.80%</td></tr>
<tr><td>Example 4</td><td>5 g</td><td>1.8 eq</td><td>2.0</td><td>98.67%</td><td>5.5 g</td></tr>
<tr><td>Example 5</td><td>3.4 g</td><td>1.8 eq</td><td>1.9</td><td>98.00%</td><td>3.9 g</td><td>96.40%</td></tr>
<tr><td>Example 6</td><td>1 g</td><td>1.0 eq</td><td>1.9</td><td>99.15%</td><td>0.8 g</td><td rowspan="2">96.50%</td></tr>
<tr><td>Comparative Example 1</td><td>1 g</td><td>3.05 eq</td><td>3.0</td><td>97.02%</td><td>1.2 g</td></tr>
</table>

As can be seen from the table above, in the salt-forming process of the free base and phosphoric acid, the purity of the product obtained was higher, and compared to the starting material, the purity was not reduced but improved by 1%, that is, the salt-forming process of the free base and phosphoric acid has a greater impurity removal effect.

When the equivalent of phosphoric acid was 1.0 eq, 1.8 eq, or 1.9 eq, the bis((N-3-aminopropyl)-(N-(3,4-dimethoxyphenylpropionyl)))aminopropylamine phosphate prepared through the reaction had an acid-base ratio of 1.9-2.1. Meanwhile, in the preparation process, the salt was easily precipitated from the reaction system, and could be separated from the system by a simple filtration operation, which is greatly conducive to industrial mass production. However, when the equivalent of phosphoric acid was 3.05 eq, the bis((N-3-aminopropyl)-(N-(3,4-dimethoxyphenylpropionyl)))aminopropylamine phosphate prepared through the reaction had an acid-base ratio of 3.0, and serious wall sticking was observed in the reaction process. When the equivalent of phosphoric acid was 1.9 eq, the reaction system was good, and the solid formed was homogeneous. When the equivalent of phosphoric acid was 3.05 eq, the mixture in the reaction system was obviously inhomogeneous, the bottom of the mixed system was whitish, the middle part of the mixed system had a slightly yellowish oil sticked to the inner wall of the flask, the phosphate could not be separated directly by a filtration method from the whole system, and slurrying with a solvent and filtration were required, while the solid obtained after filtration was extremely hygroscopic at room temperature and quickly turned into an oil, which is not conducive to industrial mass production.

**Preparation of crystal form**

**Example 7**

Bis((N-3-aminopropyl)-(N-(3,4-dimethoxyphenylpropionyl)))aminopropylamine phosphate was prepared according to the method in Example 4. 3.27 g of the bis((N-3-aminopropyl)-(N-(3,4-dimethoxyphenylpropionyl)))aminopropylamine phosphate prepared was added into 11.4 g of purified water, and then 13.3 g of methanol was slowly added to the reaction vessel. The internal temperature of the reaction vessel was adjusted to 0-10°C, and then 193.0 g of methanol was slowly added to the reaction vessel. After the addition was completed, the internal temperature of the reaction vessel was controlled to 0-10°C, and the mixture was stirred for 3-16 h to obtain a white solid. The white solid was separated by centrifugation to obtain a wet product, which was dried under vacuum at 25°C and then characterized by XRPD. It was found that the bis((N-3-aminopropyl)-(N-(3,4-dimethoxyphenylpropionyl)))aminopropylamine phosphate was in phosphate crystal form A. The XRPD pattern of the crystal form A is shown in FIG. 1. The corresponding X-ray powder diffraction pattern data is shown in Table 8, which lists the diffraction angle 2θ and relative intensity (expressed as a percentage relative to the most intense peak).

Table 8

| Angle [2θ] | D value | Relative intensity |
|---|---|---|
| 6.449 ° | 13.69497 Å | 88.2% |
| 9.681 ° | 9.12838 Å | 17.1% |
| 11.837 ° | 7.47012Å | 31.4% |
| 13.021 ° | 6.79357 Å | 36.5% |
| 14.780 ° | 5.98887 Å | 87.4% |
| 15.291 ° | 5.78988 Å | 64.0% |
| 16.227 ° | 5.45785 Å | 63.5% |
| 16.532 ° | 5.35780 Å | 40.1% |
| 16.958 ° | 5.22415 Å | 44.0% |
| 17.420 ° | 5.08677 Å | 8.1% |
| 18.404 ° | 4.81696 Å | 45.3% |
| 18.665 ° | 4.75006 Å | 71.8% |
| 19.464 ° | 4.55698 Å | 62.2% |
| 20.819 ° | 4.26325 Å | 68.8% |
| 21.937 ° | 4.04845 Å | 10.2% |
| 22.605 ° | 3.93027 Å | 89.4% |
| 22.862 ° | 3.88678 Å | 100.0% |
| 23.741 ° | 3.74480 Å | 77.3% |
| 25.149 ° | 3.53827 Å | 34.5% |

(continued)

| Angle [2θ] | D value | Relative intensity |
|---|---|---|
| 26.117 ° | 3.40918 Å | 41.2% |
| 27.008 ° | 3.29877 Å | 36.8% |
| 27.499 ° | 3.24096 Å | 26.6% |
| 29.310° | 3.04472 Å | 4.1% |
| 31.621 ° | 2.82724 Å | 2.9% |
| 32.873 ° | 2.72233 Å | 2.7% |
| 37.247 ° | 2.41208 Å | 3.8% |
| 37.931 ° | 2.37018 Å | 7.8% |

The phosphate crystal form A was characterized by TGA and DSC, and the results are shown in FIG. 2. The TGA results showed that the sample has a weight loss of 2.714% when heated from 22°C to 150°C, and the weight loss is weight loss of water desorption. The DSC results showed that the sample has an endothermic peak at 71.28°C, which is the peak of water desorption; the sample has an endothermic peak at 185.64°C, which is the peak corresponding to the crystal form transition of the sample; and the DSC heating initiation signal is presumed to be an instrument signal.

Through detection, the phosphate crystal form A prepared has an acid-base ratio of 2.0, and is slightly hygroscopic; and has good stability, showing no change in crystal form after being placed under the conditions of 25°C/60% RH, illumination, 60°C and 80°C for 2 weeks.

**Claims**

1. A bis((N-3-aminopropyl)-(N-(3,4-dimethoxyphenylpropionyl)))aminopropylamine phosphate, wherein the bis((N-3-aminopropyl)-(N-(3,4-dimethoxyphenylpropionyl)))aminopropylamine phosphate has the following structure:

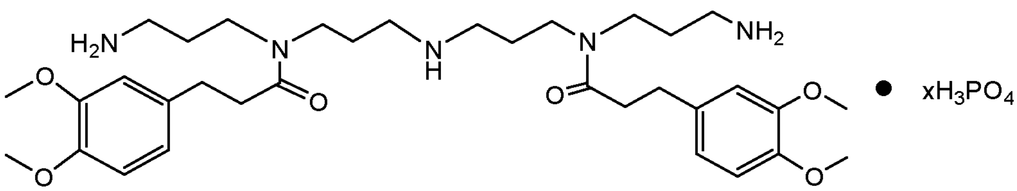

wherein x is 1-3, preferably x is 1.5-2.5, more preferably x is 1.9-2.1, and most preferably x is 2.0.

2. A preparation method for the bis((N-3-aminopropyl)-(N-(3,4-dimethoxyphenylpropionyl)))aminopropylamine phosphate according to claim 1, wherein the preparation method is: mixing bis((N-3-aminopropyl)-(N-(3,4-dimethoxyphenylpropionyl)))aminopropylamine with phosphoric acid for reaction to obtain a solid, preferably, a solvent for the reaction is selected from one or more of: ethyl acetate, dichloromethane, methanol, water, chloroform, ethanol, acetone, acetonitrile, butanol, dimethylformamide, dimethyl sulfoxide, methyl *tert-butyl* ether, isopropylamine, and isopropanol; further preferably, the solvent for the reaction is selected from one or more of: methanol, ethanol, ethyl acetate, acetone, and dichloromethane; more preferably, the solvent for the reaction is methanol or/and ethanol; preferably, the reaction is performed at a temperature of 0-35°C.

3. Crystal form A of bis((N-3-aminopropyl)-(N-(3,4-dimethoxyphenylpropionyl)))aminopropylamine phosphate, wherein the XRPD pattern of the crystal form A has characteristic peaks in at least three of positions having 2θ values of 6.4° ± 0.2°, 9.7° ± 0.2°, 16.2° ± 0.2°, 19.5° ± 0.2°, 22.9° ± 0.2°, and 26.1° ± 0.2°.

4. The crystal form A of bis((N-3-aminopropyl)-(N-(3,4-dimethoxyphenylpropionyl)))aminopropylamine phosphate according to claim 3, wherein the XRPD pattern of the crystal form A further has characteristic peaks in at least three of positions having 2θ values of 11.80° ± 0.2°, 13.0° ± 0.2°, 14.8° ± 0.2°, 15.3° ± 0.2°, 18.7° ± 0.2°, 20.8° ± 0.2°, 22.6° ± 0.2°, and 23.7° ± 0.2°.

5. The crystal form A of bis((N-3-aminopropyl)-(N-(3,4-dimethoxyphenylpropionyl)))aminopropylamine phosphate according to claim 3 or 4, wherein the crystal form A has an XRPD pattern substantially as shown in FIG. 1; preferably, the crystal form A has a DSC pattern substantially as shown in FIG. 2; preferably, the crystal form A has a TGA pattern substantially as shown in FIG. 2.

6. A preparation method for crystal form A of bis((N-3-aminopropyl)-(N-(3,4-dimethoxyphenylpropionyl)))aminopropylamine phosphate, wherein: the bis((N-3-aminopropyl)-(N-(3,4-dimethoxyphenylpropionyl)))aminopropylamine phosphate is mixed with a positive solvent and then mixed with an anti-solvent for crystallization to obtain the phosphate crystal A.

7. The preparation method for the crystal form A of bis((N-3-aminopropyl)-(N-(3,4-dimethoxyphenylpropionyl)))aminopropylamine phosphate according to claim 6, wherein the reaction is performed at a temperature of 0-30°C, preferably the reaction is performed at a temperature of 0-10°C, and more preferably the reaction is performed at a temperature of 0-5°C.

8. The preparation method for the crystal form A of bis((N-3-aminopropyl)-(N-(3,4-dimethoxyphenylpropionyl)))aminopropylamine phosphate according to claim 6, wherein the positive solvent is water, a mixed solution of methanol and water, or a mixed solution of ethanol and water; the anti-solvent is selected from one or more of: methanol, ethanol, tetrahydrofuran, ethyl acetate, or toluene.

9. Use of the bis((N-3-aminopropyl)-(N-(3,4-dimethoxyphenylpropionyl)))aminopropylamine phosphate according to claim 1 or the crystal form A of bis((N-3-aminopropyl)-(N-(3,4-dimethoxyphenylpropionyl)))aminopropylamine phosphate according to any one of claims 3-5 in preparing an anti-PAMP medicament,
wherein preferably, the PAMP is selected from one or more of: bacterial lipopolysaccharide, bacterial genome, peptidoglycan, lipoteichoic acid, viral RNA, and zymosan.

10. Use of the bis((N-3-aminopropyl)-(N-(3,4-dimethoxyphenylpropionyl)))aminopropylamine phosphate according to claim 1 or the crystal form A of bis((N-3-aminopropyl)-(N-(3,4-dimethoxyphenylpropionyl)))aminopropylamine phosphate according to any one of claims 3-5 in preparing a medicament for preventing and/or treating systemic inflammatory response syndrome or an autoimmune disease,

    preferably, the systemic inflammatory response syndrome is sepsis;
    preferably, the autoimmune disease is selected from one or more of: organ-specific autoimmune disease, systemic lupus erythematosus, rheumatoid arthritis, systemic vasculitis, scleroderma, pemphigus, dermatomyositis, mixed connective tissue disease, autoimmune hemolytic anemia, thyroid autoimmune disease, and ulcerative colitis.

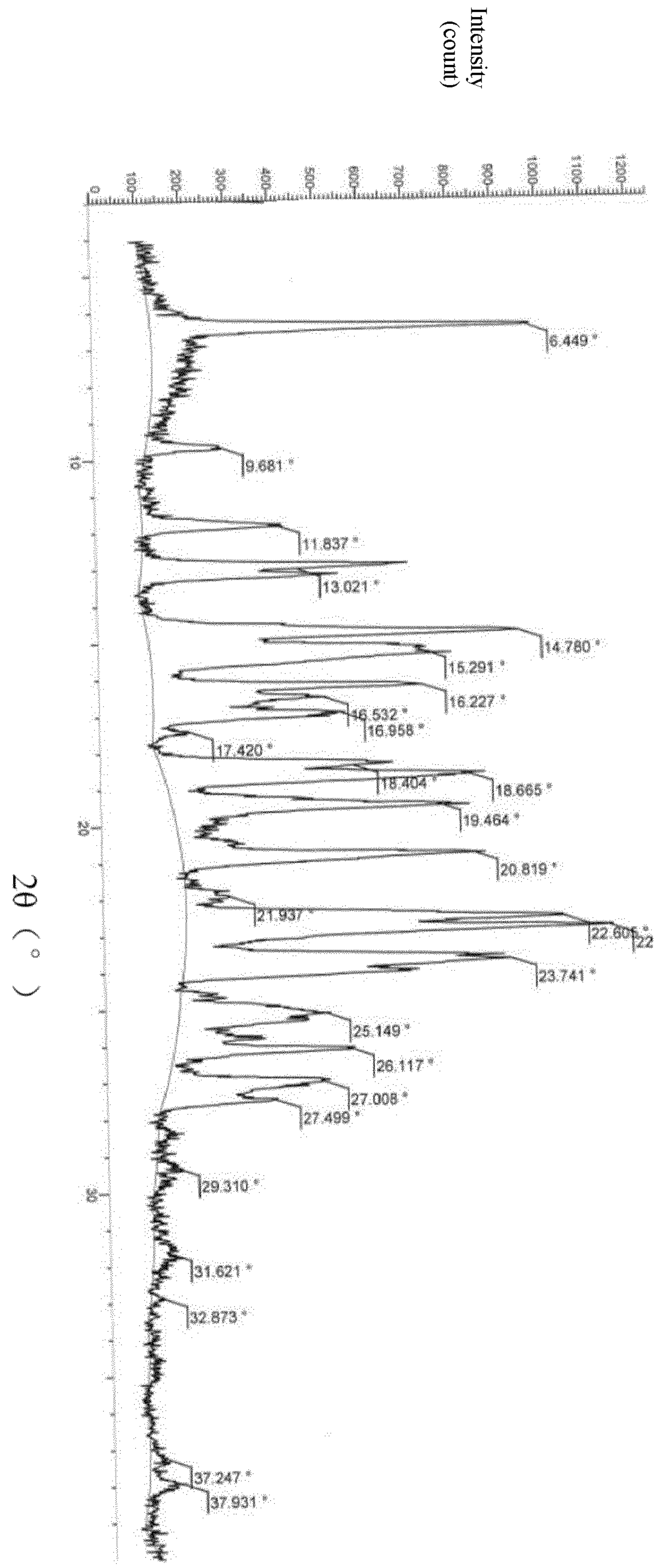

Intensity (count)
0
100
200
300
400
500
600
700
800
900
1000
1100
1200
10
20
30
2θ (°)
6.449 °
9.681 °
11.837 °
13.021 °
14.780 °
15.291 °
16.227 °
16.532 °
16.958 °
17.420 °
18.404 °
18.665 °
19.464 °
20.819 °
21.937 °
22.605 °
23.741 °
25.149 °
26.117 °
27.008 °
27.499 °
29.310 °
31.621 °
32.873 °
37.247 °
37.931 °


FIG. 1

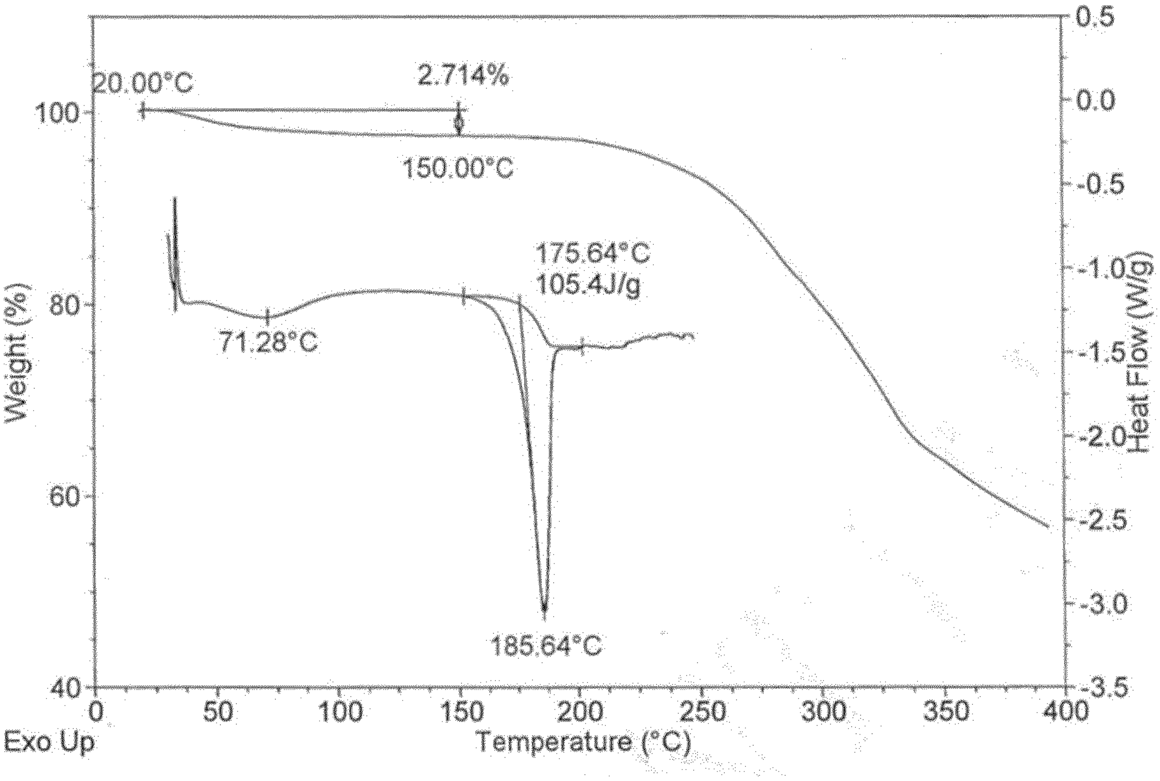
20.00°C
2.714%
150.00°C
175.64°C
105.4J/g
71.28°C
185.64°C
Weight (%)
Heat Flow (W/g)
Exo Up
Temperature (°C)
100
80
60
40
0.5
0.0
-0.5
-1.0
-1.5
-2.0
-2.5
-3.0
-3.5
0
50
100
150
200
250
300
350
400

FIG. 2

**INTERNATIONAL SEARCH REPORT**

International application No.
**PCT/CN2023/114282**

**A. CLASSIFICATION OF SUBJECT MATTER**

C07C235/34(2006.01)i; C07C231/12(2006.01)i; A61K31/165(2006.01)i; A61P29/00(2006.01)i; A61P37/00(2006.01)i

According to International Patent Classification (IPC) or to both national classification and IPC

**B. FIELDS SEARCHED**

Minimum documentation searched (classification system followed by classification symbols)

IPC: C07C235/-, C07C231/-, A61K31/-

Documentation searched other than minimum documentation to the extent that such documents are included in the fields searched

Electronic data base consulted during the international search (name of data base and, where practicable, search terms used)

CNKI, CNTXT, VEN, STN: 氨基丙基, 二甲氧, 苯丙酰基, 丙胺, 磷酸, 结晶, 晶体, aminopropyl, dimethoxy, benzenepropanamide, phosphate, CAS RN: 1883420-69-6

**C. DOCUMENTS CONSIDERED TO BE RELEVANT**

| Category* | Citation of document, with indication, where appropriate, of the relevant passages | Relevant to claim No. |
|---|---|---|
| X | WO 2017071629 A1 (CHONGQING ANTIXIN BIOTECHNOLOGY CO., LTD.) 04 May 2017 (2017-05-04)<br>description, page 4, and embodiments 1, 3, and 15 | 1-10 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 December 2023** | **11 December 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/114282**

| Patent document cited in search report | Publication date (day/month/year) | Patent family member(s) | Publication date (day/month/year) |
|---|---|---|---|
| WO 2017071629 A1 | 04 May 2017 | EP 3369725 A1 | 05 September 2018 |
| | | EP 3369725 A4 | 01 May 2019 |
| | | EP 3369725 B1 | 07 October 2020 |
| | | JP 2018534364 A | 22 November 2018 |
| | | JP 6899394 B2 | 07 July 2021 |
| | | AU 2016347239 A1 | 17 May 2018 |
| | | AU 2016347239 B2 | 15 April 2021 |
| | | BR 112018008706 A2 | 30 October 2018 |
| | | BR 112018008706 B1 | 24 January 2023 |
| | | US 2018338934 A1 | 29 November 2018 |
| | | US 10449167 B2 | 22 October 2019 |
| | | CA 3003518 A1 | 04 May 2017 |
| | | CA 3003518 C | 03 August 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 105348137 B
- CN 201510729318

### Non-patent literature cited in the description

- Phamacopoeia of the People's Republic of China. 2015